Europäisches Patentamt

(19) European Patent Office  (11) Numéro de publication: **0 163 680**

Office européen des brevets  **B1**

(12) # FASCICULE DE BREVET EUROPÉEN

(51) Int. Cl.⁴: **A 61 F 5/44,** A 61 F 13/02, A 61 F 13/18

(45) Date de publication du fascicule du brevet:
**21.09.88**

(21) Numéro de dépôt: **84904134.8**

(22) Date de dépôt: **06.11.84**

(86) Numéro de dépôt international:
**PCT/FR 84/00248**

(87) Numéro de publication internationale:
**WO 85/02110 (23.05.85 Gazette 85/12)**

(54) **ARTICLE A JETER ABSORBANT LES LIQUIDES.**

(30) Priorité: **08.11.83 FR 8317723**

(43) Date de publication de la demande:
**11.12.85 Bulletin 85/50**

(45) Mention de la délivrance du brevet:
**21.09.88 Bulletin 88/38**

(84) Etats contractants désignés:
**AT BE CH DE FR GB LI LU NL SE**

(56) Documents cité:
**FR-A-2 137 859**
**FR-A-2 470 590**
**GB-A-1 406 615**
**US-A-4 041 950**

(73) Titulaire: **KAYSERSBERG SA, Route de Lapoutroie,
F-68240 Kaysersberg (FR)**

(72) Inventeur: **PIGNEUL, Raymond, 2, rue des Vosges,
F-68320 Durrenentzen (FR)**

(74) Mandataire: **David, Daniel, KAYSERSBERG 54,
avenue Hoche, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

## Description

L'invention a pour objet un article à jeter absorbant les liquides. Cet article est destiné à être utilisé en tant que change complet pour adultes ou pour bébés mais peut également être employé comme serviette périodique pour l'absorption des liquides menstruels ou comme pansement.

Outre la fonction d'absorber les liquides, un tel article doit éviter de conserver le liquide en contact avec la peau de l'utilisateur afin, d'une part d'améliorer le confort général de celui-ci et plus particulièrement d'empêcher, autant que possible, que le liquide, qui subit un phénomène de dégradation appelé macération, ne provoque des irritations de la peau. Pour cela, il est nécessaire que le liquide migre vers les zones du matelas éloignées de la peau de l'utilisateur.

Il a été ainsi proposé dans les brevets français n° 1.326.227 et 2.301.189 un article à jeter constitué d'un matelas absorbant en mousse de cellulose recouvert sur la face externe d'une feuille de polyéthylène et sur la face interne d'une feuille de non tissé, l'ensemble étant replié selon des lignes longitudinales pour former un soufflet. Les différentes couches ainsi formées sont soudées entre elles au niveau de leur zone médiane tandis que les extrémités peuvent se déployer pour entourer la taille de l'utilisateur.

Un tel dispositif présente, toutefois, l'inconvénient suivant:

Lors de son émission, le liquide ne rencontre qu'une seule épaisseur de mousse de cellulose au niveau de la partie centrale, car les différentes épaisseurs ne peuvent être traversées, du fait de la présence de la feuille imperméable. Il en est de même au niveau des parties qui sont situées à l'avant et à l'arrière de l'utilisateur, car le change, en position, est déployé.

Afin de remédier à cet inconvénient, le brevet US 3 746 592 décrit un matelas absorbant plié en forme de C présentant deux couches superposées et un canal central. Ce dispositif permet une meilleure répartition de l'urine, mais présente deux désavantages majeurs.

Le matelas a une épaisseur constante importante sur toute sa surface, ce qui rend difficile son adaptation à l'anatomie de l'utilisateur. En effet, il est préférable que les zones destinées à entourer le haut des cuisses et qui sont souvent pourvues de laminettes élastiques présentent une certaine souplesse.

En outre, le liquide est "stocké" dans une zone trop proche de la peau de l'utilisateur. Certes, le liquide est pour une partie drainé vers la couche externe, mais il a également tendance à remonter au niveau de la couche interne, ce qui a pour conséquence de considérablement diminuer l'effet recherché.

Le brevet US 4 041 950 décrit une couche dont le matelas absorbant est replié sur lui-même, de manière à ménager des bourrelets prolongés par des extensions latérales. Toutefois, ces bourrelets servent seulement à éviter les écoulements latéraux, et ne peuvent éviter que la zone centrale dans laquelle se rassemblent les liquides ne vienne au contact de la peau de l'utilisateur.

L'invention a pour objet de remédier à ces inconvénients en proposant un article absorbant présentant une absorption améliorée tout en assurant une très bonne adaptation à l'anatomie de l'utilisateur.

Selon l'invention, l'article absorbant comporte un matelas absorbant de forme sensiblement rectangulaire recouvert sur sa face interne d'une feuille perméable aux liquides telle qu'une feuille de non tissé et sur sa face externe d'une feuille imperméable aux liquides telles qu'une feuille de polyéthylène, ces deux feuilles étant partiellement ou totalement soudées entre elles le long de leurs bords, et est caractérisé en ce que le matelas absorbant est formé à l'intérieur de l'enveloppe définie par les feuilles d'un ensemble d'au moins trois couches superposées, constituées d'une ou plusieurs nappes de mousse de cellulose, la couche externe étant continue tandis que les autres couches sont constituées de deux demi-couches juxtaposées et disposées symétriquement par rapport à l'axe longitudinal du matelas, de manière à définir un canal de drainage des liquides vers la couche externe qui reste éloignée de la peau de l'utilisateur, la couche interne étant prolongée au-delà des bords longitudinaux de la couche externe afin de former deux extensions latérales.

Dans cette description, le qualificatif "interne" désigne la face, ou la couche, la plus proche de la peau de l'utilisateur tandis que le qualificatif "externe" désigne la face, ou la couche, la plus éloignée de la peau de l'utilisateur.

Le canal central formé par la juxtaposition des demi-couches permet le drainage du liquide vers la couche externe et ainsi assure le stockage du liquide loin de la peau de l'utilisateur, dans la couche externe.

Il est préférable, à la fois pour des raisons de fabrication et de capacité d'absorption, que le matelas absorbant soit formé d'une seule nappe et comporte une bande centrale longitudinale correspondant à la couche externe et au moins deux bandes latérales situées de part et d'autre de la bande centrale et repliées sur la face interne de celle-ci en zigzag (en accordéon) de manière à former les autres couches.

Pour des raisons qui seront expliquées ci-dessous, il est préférable que le matelas soit constitué d'un nombre impair de couches superposées. Avantageusement ce matelas sera constitué de trois couches superposées: cette forme préférée de l'invention permet d'obtenir à la fois une très bonne absorption, une distance suffisante entre la peau de l'utilisateur et l'endroit où le liquide est stocké, et un très bon confort.

Il est également souhaitable que les bords longitudinaux de la couche interne, dans le cas où le matelas absorbant comporte un nombre impair de couches, soient prolongés au-delà des bords longitudinaux de la couche externe afin de former deux extensions latérales monocouches. La présence, au niveau des bords longitudinaux, d'une seule couche permet une meilleure adaptation à l'anatomie de l'utilisateur. Dans ce cas, le matelas comporte une zone centrale longitudinale à plusieurs épaisseurs et des zones latérales longitudinales monocouches.

Les avantages énumérés ci-dessus sont encore accentués par la présence, dans la couche externe, de grains de "superabsorbant" au moins dans la zone de réception du fluide. Les grains de "superabsorbant" sont des polyélectrolytes hydrocolloïdaux insolubles dans l'eau et absorbant plusieurs fois leur poids en liquide aqueux. Ces composés sont par exemple décrits dans le brevet américain US 4 043 952. Avantageusement, la couche externe sera formée de deux nappes superposées et les grains de superabsorbant seront insérés entre ces deux nappes.

Il est essentiel, afin d'assurer une migration optimum du liquide que seule la couche externe comporte ces grains de superabsorbant.

L'invention sera mieux comprise en se référant à un mode de réalisation de l'art antérieur (brevet US 3 746 592) et à quatre modes de réalisation particuliers selon la présente demande. Ces modes de réalisation sont représentés par les dessins en annexe.

La figure 1 est une vue en coupe transversale, d'un premier type de matelas absorbant déplié, ne comportant pas de grains de superabsorbant.

La figure 2 est une vue en plan de dessus du matelas absorbant tel qu'illustré à la figure 1.

La figure 3 est une vue en coupe transversale du même matelas absorbant que celui illustré à la figure 1, mais replié en trois couches superposées.

La figure 4 est une vue en coupe transversale d'un article absorbant comportant le même matelas absorbant que celui illustré à la figure 3, compacté.

La figure 5 est une vue en coupe transversale d'un second type de matelas absorbant déplié, ne comportant pas de grains de superabsorbant.

La figure 6 est une vue en plan de dessus du matelas absorbant illustré à la figure 5.

La figure 7 est une vue en coupe transversale du même matelas absorbant que celui illustré à la figure 5 mais replié en trois couches superposées, la couche interne étant prolongée par des extensions latérales monocouches.

La figure 8 est une vue en coupe transversale d'un article absorbant comportant le matelas absorbant illustré à la figure 7, compacté.

La figure 9 est une vue en coupe transversale d'un troisième type de matelas absorbant replié, incorporé dans l'article absorbant correspondant. Afin de ne pas alourdir inutilement la description, le matelas absorbant, contrairement aux deux types de réalisations précédentes, n'a pas été représenté déplié en coupe transversale. Le lecteur pourra se reporter pour mémoire aux figures 5, 6.

La figure 10 est une vue en plan de dessus du matelas absorbant de la figure 9.

La figure 11 est une vue en coupe transversale d'un quatrième type de matelas absorbant non compacté déjà incorporé dans l'article absorbant correspondant. La même remarque que pour le matelas absorbant illustré à la figure 9 s'applique.

La figure 12 est une vue en coupe transversale d'un matelas absorbant décrit dans le brevet US 3 746 592.

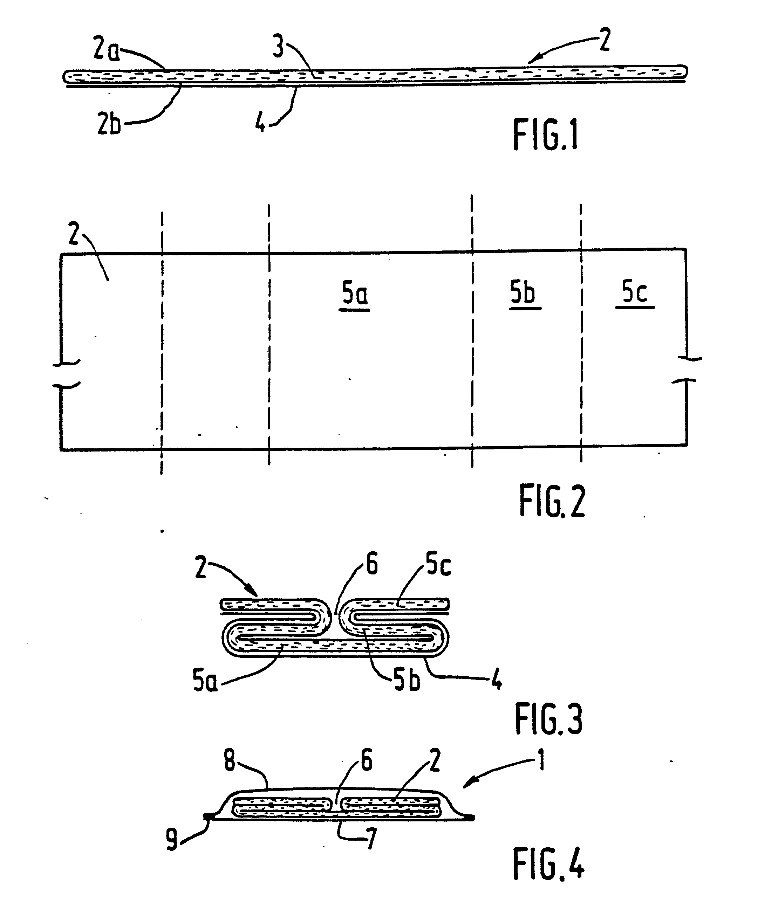

Selon les figures 1, 2, 3, 4, l'article 1 est formé d'un matelas absorbant 2 rectangulaire ayant une face interne 2a et une face externe 2b. Le matelas absorbant 2 est constitué d'une nappe de mousse de cellulose 3 supportée par une feuille de ouate de celulose 4.

Le matelas absorbant 2 est partagé en cinq bandes longitudinales:
- une bande centrale 5a dont la face externe est destinée à former la face externe du matelas absorbant 2 une fois celui-ci replié.
- deux bandes intermédiaires 5b de part et d'autre de la bande centrale 5a, de largeur égale à la moitié de la largeur de la bande centrale 5a.
- deux bandes latérales 5c formant les extrémités longitudinales du matelas absorbant 2 dont les faces internes sont destinées à former la face interne de ce matelas. La largeur des bandes 5c est égale à celles des bandes 5b.

Lorsque les bandes 5b et 5c sont repliées en zigzag sur la face interne de la bande 5a, un canal central longitudinal 6 est formé.

Le matelas absorbant replié est ensuite recouvert sur sa face externe d'une feuille de polyéthylène 7 et sur sa face interne d'une feuille de non tissé 8, les deux feuilles étant soudées 9 tout au long de leurs bords.

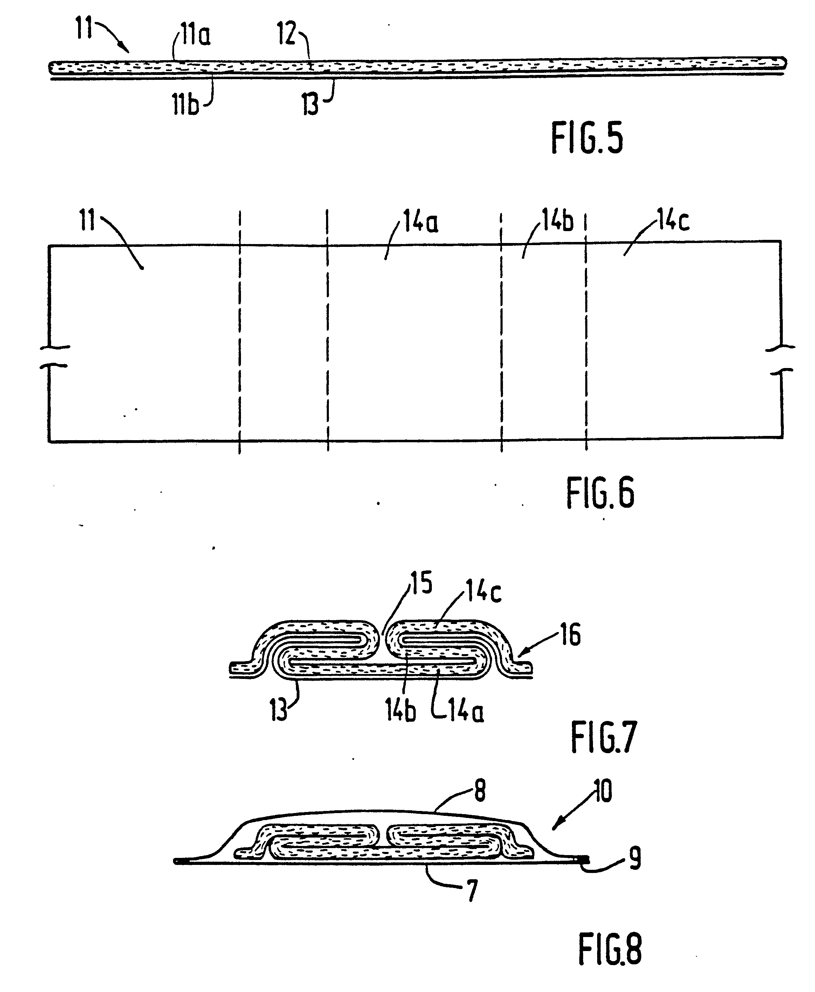

Selon les figures 5, 6, 7, 8, l'article 10 est formé d'un matelas absorbant 11 rectangulaire ayant une face interne 11a et une face externe 11b. Le matelas absorbant 11 est constitué d'une nappe de mousse de cellulose 12, supportée par une feuille de ouate de cellulose 13.

Le matelas absorbant 11 est partagé en cinq bandes longitudinales:
- une bande centrale 14a dont la face externe est destinée à former la face externe du matelas absorbant 11, une fois celui-ci replié.
- deux bandes intermédiaires 14b, de part et d'autre de la bande centrale 14a, de largeur égale à la moitié de la largeur de la bande centrale 14a.
- deux bandes latérales 14c formant les extrémités longitudinales du matelas absorbant 11, de largeur supérieure à celle des bandes 14b, dont les faces internes sont destinées à former la face interne de ce matelas, une fois celui-ci replié.

Lorsque les bandes 14b et 14c sont repliées en zigzag sur la face interne de la bande 14a, un canal central longitudinal 15 est formé ainsi que deux extensions longitudinales monocouches 16.

Le matelas absorbant replié est ensuite recouvert sur sa face externe d'une feuille de polyéthylène 7 et sur sa face interne d'une feuille de non tissé 8, les deux feuilles étant soudées 9 tout au long de leurs bords.

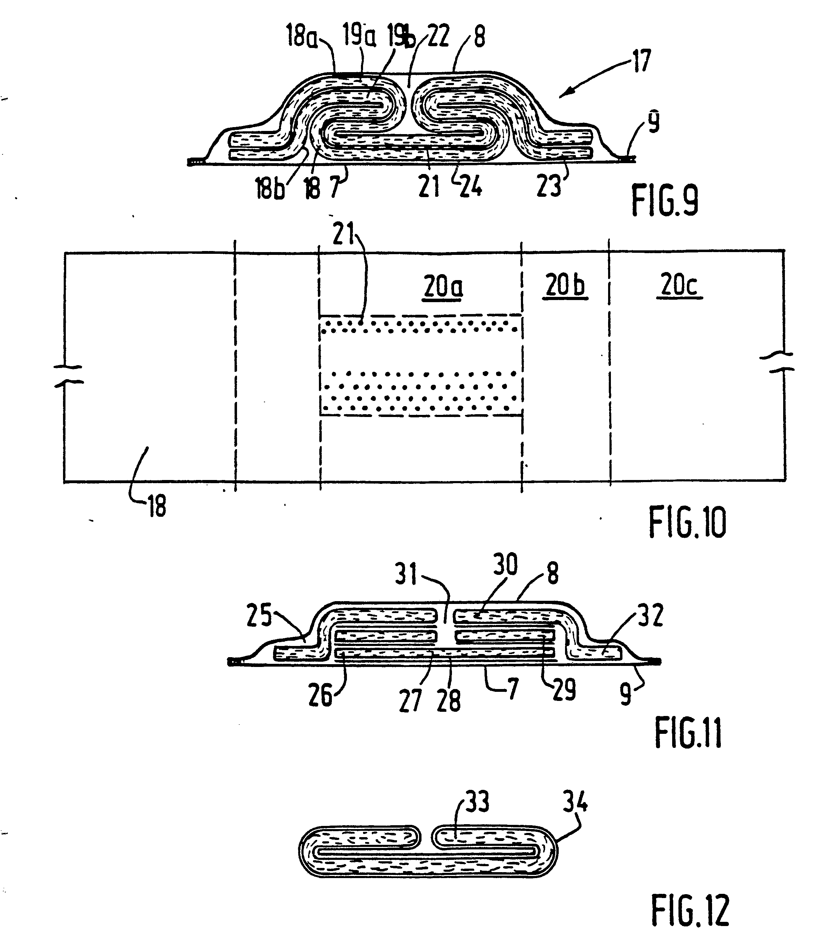

Selon les figures 9 et 10, l'article 17 est formé d'un matelas absorbant 18 rectangulaire ayant une face interne 18a et une face externe 18b, constitué de deux nappes rectangulaires 19a et 19b superposées, en mousse de cellulose supportée par une feuille de ouate de cellulose 24.

Le matelas absorbant 18 est partagé en cinq bandes longitudinales:
- une bande centrale 20a dont la face externe est destinée à former la face externe du matelas absorbant 18 et dans la zone médiane de laquelle, entre les deux nappes 19a et 19b, sont déposés des grains de superabsorbant 21.
- deux bandes intermédiaires 20b situées de part et d'autre de la bande centrale 20a et de largeur égale à la moitié de celle-ci.
- deux bandes latérales 20c formant les extrémités du matelas absorbant 18, de largeur supérieure à celle des bandes 20b et dont les faces internes sont destinées à former la face interne de ce matelas absorbant.

Lorsque les bandes sont repliées en zigzag sur la face interne de la bande 20a, un canal central 22 est formé, ainsi que deux extensions longitudinales monocouches 23.

Le matelas absorbant 18 replié, est ensuite recouvert sur sa face externe d'une feuille de polyéthylène 7 et sur sa face interne d'une feuille de non tissé 8, les deux feuilles étant soudées 9 tout au long de leurs bords.

Selon la figure 11, l'article est formé d'un matelas absorbant 25 constitué d'une couche externe 26 formée d'une nappe de mousse de cellulose 27 supportée par une feuille de ouate de cellulose 28 et de deux paires de demi-couches superposées 29 et 30, disposées sur la face interne de la couche externe 26 de part et d'autre de l'axe longitudinal de cette dernière, chacune des demi-couches étant supportée par une feuille de ouate de cellulose. Les demi-couches juxtaposées forment ainsi un canal de drainage 31. Par ailleurs, les demi-couches internes 30 présentent des extensions 32 formant des bandes longitudinales extérieures. Comme précédemment, le matelas absorbant est recouvert d'une feuille de non tissé 8 et d'une feuille de polyéthylène 7 soudées 9 le long de leurs bords.

A titre de comparaison est illustré, figure 12, un matelas absorbant en forme C formé d'une nappe de mousse de cellulose 33 enveloppée d'une feuille de ouate de cellulose 34.

**Revendications**

1. Article à jeter (10) absorbant les liquides, comportant un matelas absorbant (11) de forme sensiblement rectangulaire recouvert sur sa face interne d'une feuille (8), perméable aux liquides, telle qu'une feuille de non tissé et sur sa face externe d'une feuille (7), imperméable aux liquides, telle qu'une feuille de polyéthylène, ces deux feuilles étant partiellement ou totalement soudées entre elles le long de leurs bords et caractérisé en ce que le matelas (11) est formé à l'intérieur de l'enveloppe définie par les feuilles (7) et (8) d'un ensemble d'au moins trois couches (14a, 14b, 14c) superposées, constituées d'une ou plusieurs nappes de mousse de cellulose, la couche externe (14a) étant continue tandis que les autres couches (14b, 14c) sont constituées de deux demi-couches juxtaposées et disposées symétriquement par rapport à l'axe longitudinal du matelas (11), de manière à définir un canal de drainage des liquides vers la couche externe qui reste éloignée de la peau de l'utilisateur, la couche internel (14c) étant prolongée au-delà des bords longitudinaux de la couche externe afin de former deux extensions latérales.

2. Article à jeter selon la revendication 1, caractérisé en ce que le matelas absorbant (11) est formé d'une seule nappe et comporte une bande centrale (14a) longitudinale correspondant à la couche externe (14a) et d'au moins deux bandes latérales (14b, 14c) de part et d'autre de la bande centrale, lesquelles sont repliées en zigzag sur la face interne de la bande centrale (14a) de manière à former les autres couches.

3. Article à jeter selon la revendication 1 ou 2, caractérisé en ce que le matelas (11) est constitué d'un nombre impair de couches superposées.

4. Article à jeter selon la revendication 3, caractérisé en ce que le matelas (11) est constitué de trois couches superposées.

5. Article à jeter selon l'une des revendications précédentes, caractérisé en ce que les différentes couches sont supportées par une feuille de ouate de cellulose (13).

6. Article à jeter selon l'une des revendications précédentes, caractérisé en ce que la couche externe (14a) comporte au moins dans la zone de réception des fluides des grains de superabsorbant (21).

**Patentansprüche**

1. Wegwerfartikel (10) zur Absorption von Flüssigkeiten, bestehend aus einem flüssigkeitsabsorbierenden Kissen (11) mit deutlich rechteckiger Form, dessen innere Fläche mit einer flüssigkeitsdurchlässigen nicht gewebten Folie (8) bedeckt ist und dessen äußere Fläche von einer flüssigkeitsundurchlässigen Folie (7), z.B. aus Polyethylen, bedeckt wird, wobei die beiden Folien über einen Teil oder die gesamte Länge ihrer Kanten miteinander verschweißt sind, dadurch gekennzeichnet, daß das Kissen (11) im Inneren eines durch die Folien (7) und (8) definierten Umschlages angeordnet ist und durch mindestens drei übereinanderliegende Schichten (14a, 14b, 14c) aufgebaut wird, die aus einem oder mehreren Zellstoffstreifen bestehen, wobei die äußere Schicht (14a) durchgängig ist, während die anderen Schichten (14b, 14c) aus zwei nebeneinander liegenden, symmetrisch zur Längsachse des Kissens (11) angeordneten Halbschichten bestehen, wodurch ein Entwässerungskanal gebildet wird, der zur äußeren von der Haut des Benutzers entfernten Schicht führt, und wobei die innere Schicht (14c) über die Längsränder der äußeren Schicht hinweg verlängert werden kann, um seitliche Vergrößerungen zu bilden.

2. Wegwerfartikel nach Anspruch 1, dadurch gekennzeichnet, daß das flüssigkeitsabsorbierende Kissen (11) aus einem einzigen Streifen mit einem zentralen länglichen Teil (14a) besteht, der der äußeren Schicht (14a) entspricht und aus mindestens zwei seitlichen Teilen (14b, 14c) auf jeder Seite des zentralen Teiles die zickzack-förmig auf die innere Fläche des zentralen Teiles (14a) gefaltet sind und so die anderen Schichten formen.

3. Wegwerfartikel nach einem der Ansprüche 1 oder 2, dadurch gekennzeichnet, daß das flüssigkeitsabsorbierende Kissen (11) aus einer ungeraden Anzahl von übereinanderliegenden Schichten aufgebaut ist.

4. Wegwerfartikel nach Anspruch 3, dadurch gekennzeichnet, daß das flüssigkeitsabsorbierende Kissen (11) aus drei übereinanderliegenden Schichten aufgebaut ist.

5. Wegwerfartikel nach einem der vorhergehenden Ansprüche, dadurch gekennzeichnet, daß die unterschiedlichen Schichten durch eine Folie aus Zellstoffwatte (13) gestützt werden.

6. Wegwerfartikel nach einem der vorhergehenden Ansprüche, dadurch gekennzeichnet, daß die äußere Schicht (14a) zumindestens in dem Bereich, wo sie die Flüssigkeit empfängt, stark absorbierende Partikel (21) enthält.

**Claims**

1. Disposable, liquid-absorbent article (10), including an absorbent pad (11) of substantially rectangular shape, covered on its inner surface with a liquid-permeable sheet (8), such as a sheet of non-woven tissue, and on its outer surface with a liquid-impermeable sheet (7), such as a sheet of polyethylene, these two sheets being partially or totally welded together along their edges, and characterised in that the pad (11) is formed within the envelope defined by the sheets (7) and (8) of an assembly of at least three superimposed layers (14a, 14b, 14c), consisting of one or more sheets of cellulose foam, the outer layer (14a) being continuous while the other layers (14b, 14c) consist of two semilayers which are juxtaposed and arranged symmetrically relative to the longitudinal axis of the pad (11), as to define a drainage channel for liquids towards the outer layer which remains distant from the user's skin, the inner layer (14c) being extended beyond the longitudinal edges of the outer layer in order to form two lateral extensions.

2. Disposable article in accordance with claim 1, characterised in that the absorbent pad (11) is formed of a single sheet and includes a central longitudinal strip (14a) corresponding to the outer layer (14a) and at least two lateral strips (14b, 14c) on either side of the central strip, which are folded in a zigzag on the inner surface of the central strip (14a) so as to form the other layers.

3. Disposable article in accordance with claim 1 or 2, characterised in that the pad (11) consists of an odd number of superimposed layers.

4. Disposable article in accordance with claim 3, characterised in that the pad (11) consists of three superimposed layers.

5. Disposable article in accordance with one of the preceding claims, characterised in that the different layers are supported by a sheet of cellulose wadding (13).

6. Disposable article in accordance with one of the preceding claims, characterised in that the outer layer (14a) includes superabsorbent granules (21) at least in the fluid reception zone.

FIG.1

FIG.2

FIG.3

FIG.4

FIG.5

FIG.6

FIG.7

FIG.8

FIG.9

FIG.10

FIG.11

FIG.12